# EUROPEAN PATENT SPECIFICATION

(11) **EP 1 414 787 B1**
(45) Date of publication and mention of the grant of the patent: **16.04.2008**
(21) Application number: 02745720.9
(22) Date of filing: 12.07.2002
(51) Int. Cl.: C07C 233/25, C07C 233/26, C07C 233/27, C07C 233/60, C07C 235/16, C07C 237/00, C07C 255/54, C07D 207/06, C07D 265/30, A01N 37/30, A01N 37/24, A01N 37/22, A01N 37/32

(54) **NEW DERIVATIVES OF SUBSTITUTED ANILINES WITH HERBICIDAL ACTIVITY**
NEUE SUBSTITUIERTE ANILINDERIVATE MIT HERBIZIDER WIRKUNG
NOUVEAUX DERIVES D'ANILINES SUBSTITUEES AYANT UNE ACTIVITE HERBICIDE

(30) Priority: 13.07.2001 IT MI20011497
(43) Date of publication of application: 06.05.2004
(73) Proprietor: Isagro Ricerca S.r.l., 20153 Milano (IT)
(72) Inventor: BETTARINI, Franco, I-28100 Novara (IT); MEAZZA, Giovanni, I-21047 Saronno-Varesse (IT); LA PORTA, Piero DI, deceased (IT); PORTOSO, Domenico, I-26900 Lodi (IT); FORNARA, Luca, I-20070 Cerro al Lambro-Milan (IT)
(74) Representative: De Gregori, Antonella
(86) International application number: PCT/IB2002/002790
(87) International publication number: WO 2003/006422

(56) References cited:
- EP-A- 0 136 678
- WO-A-98/03500
- WO-A-99/48859
- DE-A- 2 855 699
- ALEXEY V. KALININ ET AL: "The Directed Ortho Metalation-Ullmann Connection. A New Cu(I)-Catalyzed Variant for the Synthesis of Substituted Diaryl Ethers" J.ORG.CHEM., vol. 64, no. 9, 1999, pages 2986-2987, XP002219431
- ANTHONY J. PEARSON: "Studies on Selective Nucleophilic Substitution Reactions of [(Cyclopentadienyl)(1,3-dichlorobenzene)M] +PF6- Complexes (M=Fe,Ru)" J.ORG.CHEM., vol. 57, no. 13, 1992, pages 3583-3589, XP002219432
- ARTHUR W. H. WARDROP: "Preparation of Some Dibenz[b,f][1,4]oxazepines and Dibenz[b,e]azepines" J.CHEM.SOC. PERKIN I, 1976, pages 1279-1285, XP001119669
- DATABASE CAPLUS [Online] CHEMICAL ABSTRACTS SERVICE, COLUMBUS, OHIO, US; JP62167706 A, 20 January 1986 (1986-01-20) DAICEL CHEMICAL INDUSTRIES, LTD: "Synergistic,wide-spectrum herbicides containing dihydrooxopyridinecarboxamides and propionanilides" Database accession no. 107:231441 XP002219433
- DATABASE CAPLUS [Online] CHEMICAL ABSTRACTS SERVICE, COLUMBUS, OHIO, US; HARRY L. YALE ET AL.: "Novel polycyclic heterocycles. Derivatives of 5,11-dihydrodibenz[b,e][1,4]oxazepine and 5,11-dihydrodibenzo[b,e][1,4]thiazepine" Database accession no. 73:45482 XP002219434
- DATABASE CAPLUS [Online] CHEMICAL ABSTRACTS SERVICE, COLUMBUS, OHIO, US; JP48041536 B, 30 December 1970 (1970-12-30) ISHIHARA MINING AND CHEMICAL CO., LTD.: "Herbicidal compositions containing methyl phenoxycarbanylates" Database accession no. 83:73474 XP002219435
- DATABASE CAPLUS [Online] CHEMICAL ABSTRACTS SERVICE, COLUMBUS, OHIO, US; JP49133525 A, 27 April 1973 (1973-04-27) ISHIHARA MINING AND CHEMICAL CO., LTD.: "N-[2-(3-Trifluoromethylphenoxy)phenyl]car bamates as herbicides" Database accession no. 83:73484 XP002219436 -& JP 49 133525 A (ISHIHARA MINING AND CHEMICAL CO., LTD.) 27 April 1973 (1973-04-27)

## Description

The present invention relates to new derivatives of substituted anilines.

More specifically, the present invention relates to new derivatives of ortho-substituted anilines having a high herbicidal activity, the processes for its preparation and their use as herbicides for controlling weeds in agricultural crops.

Derivatives of meta-substituted anilines with a herbicidal activity are described in German patent application 2,855,699. These compounds however do not appear to be satisfactory as herbicides in that they require high applicative dosages.

Heterocyclyl- or phenyl-carboxanilides substituted, in "ortho" position with respect to the amine group, by variously substituted phenoxy, phenoxymethyl or phenylmethoxy groups, are described in patent application WO 98/03500. The products of the above patent application are essentially active as fungicides and insecticides.

The applicant has now found that a series of new derivatives of anilines substituted, in "ortho" position with respect to the amine group, by phenoxymethyl, phenylmethoxy or phenoxy groups, in turn substituted by particular groupings, have, on the contrary, a surprisingly high herbicidal activity, combined with a low phytotoxicity for one or more cultivations of agrarian interest.

The object of the present invention therefore relates to new compounds having general formula (I): 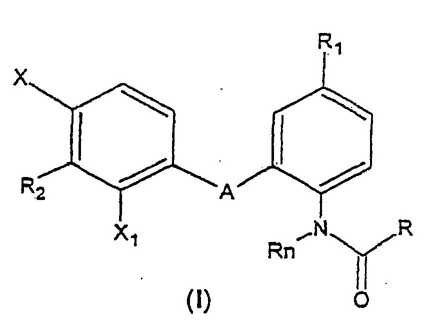 wherein:
- R represents a hydrogen atom, a C₁-C₈ haloalkyl group, a C₂-C₈ alkoxyalkyl or haloalkoxyalkyl group, a C₂-C₈ alkenyl or haloalkenyl group, a C₂-C₈ alkynyl or haloalkynyl group, a C₃-C₈ cycloalkyl or C₄-C₉ cycloalkylalkyl group optionally substituted by halogen atoms and/or C₁-C₄ alkyl or haloalkyl groups, an NRₐR_{b} group;
- Rₐ and R_{b}, the same or different, represent: a hydrogen atom; a C₁-C₈ alkyl group; a phenyl group or a benzyl group optionally substituted by halogen atoms, by CN groups, NO₂ groups, C₁-C₄ alkyl, haloalkyl, alkoxyl, haloalkoxyl groups; or Rₐ and R_{b} jointly represent a C₂-C₈ alkylene chain optionally interrupted by oxygen atoms;
- Rₙ represents a hydrogen atom, or a C₁-C₄ alkyl or haloalkyl group;
- R₁ represents a halogen atom, a C₁-C₄ alkyl or haloalkyl group, a C₁-C₄ alkoxyl or haloalkoxyl group, a C₁-C₄ alkylthio or haloalkylthio group, a cyano group, a nitro group;
- R₂ represents a C₁-C₄ haloalkyl, or haloalkoxyl group;
- X and X₁, the same or different, represent a hydrogen atom or a halogen atom;
- A represents a -CR₃R₄O- group, wherein R₃ and R₄, the same or different, represent a hydrogen atom or a C₁-C₄ alkyl, haloalkyl group.

Specific, examples of compounds having general formula (I) which are of interest for their activity are specified in Table 1:

**Table 1**

| X | X₁ | R₂ | A | R₁ | Rₙ | R |
|---|---|---|---|---|---|---|
| H | H | CF₃ | CH₂O | CH₃ | H | cyclopropyl |
| H | H | CF₃ | CH₂O | CH₃ | H | ClCH₂ |
| H | H | CF₃ | CH₂O | CH₃ | H | H |
| H | H | OCF₂H | CH₂O | CH₃ | H | cyclopropyl |
| H | H | CF₃ | CH₂O | CH₃ | H | 1-methylcyclopropyl |
| H | H | CF₃ | CH₂O | CH₃ | H | cyclobutyl |
| H | H | CF₃ | CH₂O | CH₃ | H | cyclopentyl |
| H | H | CF₃ | CH₂O | CH₃ | H | CH₃OCH₂ |
| H | H | CF₃ | CH₂O | CH₃ | H | (CH₃)₂N |
| H | H | CF₃ | CH₂O | CH₃ | H | 1-pyrrolidyl |
| H | H | CF₃ | CH₂O | CH₃ | H | 1-morpholyl |
| H | H | CF₃ | CH₂O | F | H | cyclopropyl |
| H | H | CF₃ | CH₂O | CH₃ | H | CH₃O |
| H | H | CF₃ | CH₂O | CH₃ | H | CH₃CH₂O |
| H | F | CF₃ | CH₂O | CH₃ | H | cyclopropyl |

A further object of the present invention relates to processes for the preparation of the compounds having general formula (I) .

The compounds having general formula (I) can be prepared by the reaction of an amine derivative having general formula (II) with a compound having general formula (III), according to reaction scheme 1:

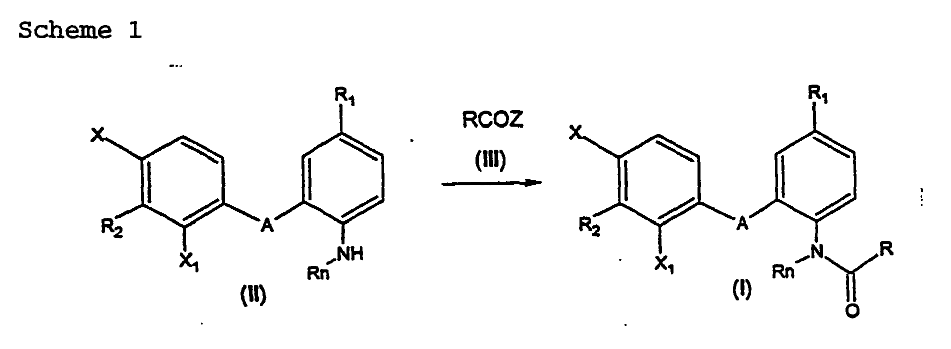

In said formulae, R, Rₙ, R₁, R₂, X, X₁ and A have the meanings described above, Z represents a halogen atom, an alkoxyl group, a hydroxyl group.

The reaction conditions for carrying out the above process can also vary in relation to the nature of the compound having formula (III).

For example, when Z represents a halogen atom, the reaction is preferably carried out in the presence of an inert solvent and in the presence of an organic or inorganic base, at a temperature ranging from -20°C to the boiling point of the reaction mixture.

Examples of solvents which can be used for the above reaction include water, aliphatic or cycloaliphatic hydrocarbons (petroleum ether, hexane, cyclohexane, etc.), chlorinated hydrocarbons (methylene chloride, chloroform, carbon tetrachloride, dichloroethane, etc.), aromatic hydrocarbons (benzene, toluene, xylene, chlorobenzene, etc.), ethers (diethyl ether, diisopropyl ether, dimethoxyethane, dioxane, tetrahydrofuran, etc.), esters (ethyl acetate, etc.), ketones (acetone, methylethylketone, methylpropyl-ketone, methylisobutyl ketone, etc.), nitriles (acetonitrile, benzonitrile, etc.), aprotic dipolar solvents (dimethylformamide, dimethylacetamide, hexamethylphosphorotriamide, dimethylsulfoxide, sulfolane, N-methylpyrrolidone, etc.).

Inorganic bases which can be used for the purpose are, for example, hydroxides, sodium and potassium carbonates and bicarbonates.

Organic bases which can be used for the purpose are, for example, triethylamine, pyridine, 4-N,N-dimethylaminopyridine, N,N-dimethylaniline, N-methylpiperidine, lutidine, diazabicyclooctane (DABCO), diazabicyclononene (DBN), diazabicycloundecene (DBU).

Alternatively, the compounds having general formula (I) wherein A represents a -CR₃R₄O- group, can be prepared by the condensation of a derivative having general formula (IV) with a phenol having general formula (V), to give an ether having general formula (Ia) according to reaction scheme 2:

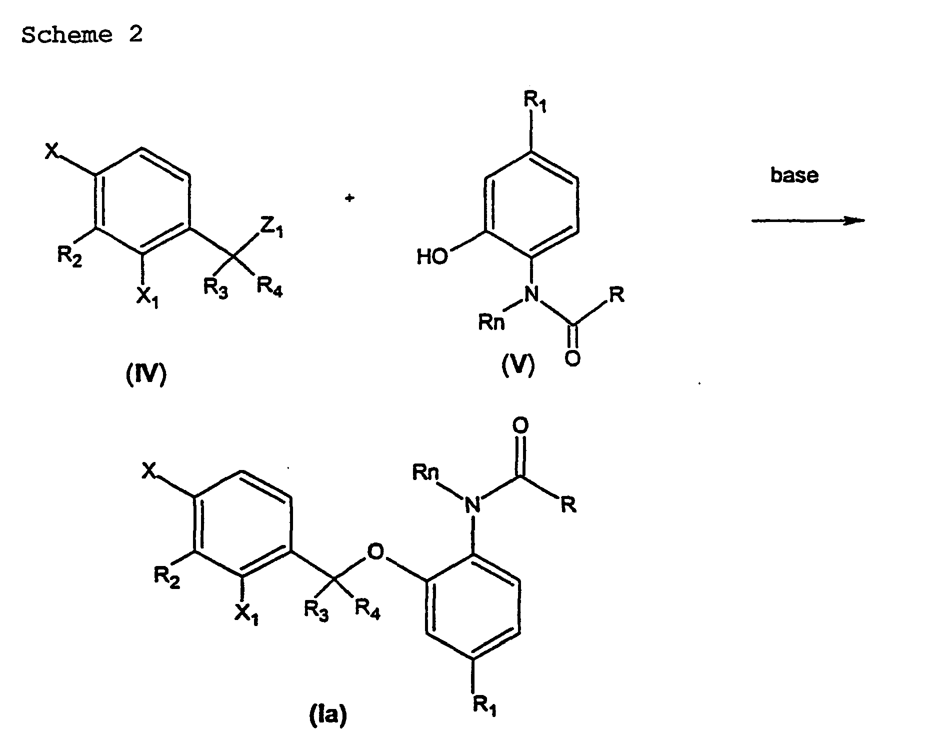

In the general formulae specified in this reaction scheme, R, Rₙ, R₁, R₂, R₃, R₄, X and X₁ have the meanings previously defined, Z₁ represents a halogen atom, preferably chlorine or bromine, or an R_{z}SO₃ group wherein R_{z} represents a C₁-C₄ alkyl group or a phenyl group optionally substituted by C₁-C₄ alkyl groups.

The etherification reaction is preferably carried out in the presence of one or more inert organic solvents and in the presence of a base, preferably inorganic, at a temperature ranging from -10°C to the boiling point of the reaction mixture.

Organic solvents which can be used for the purpose are, for example, aromatic hydrocarbons (benzene, toluene, xylene, chlorobenzene, etc.), ethers (diethyl ether, diisopropyl ether, dimethoxyethane, dioxane, tetrahydrofuran, etc.), alcohols and glycols (methanol, ethanol, methycellosolve, ethylene glycol, etc.), ketones (acetone, methylethylketone, methylpropylketone, methylisobutylketone, etc.), nitriles (acetonitrile, benzonitrile, etc.), aprotic dipolar solvents (dimethylformamide, dimethylacetamide, hexamethylphosphorotriamide, dimethylsulfoxide, sulfolane, N-methylpyrrolidone, etc.).

Inorganic bases useful for the purpose are, for example, sodium or potassium hydrides, hydroxides and carbonates.

The reaction can be advantageously carried out in a biphasic system using water and an organic solvent immiscible therewith, as solvents, in the presence of phase transfer catalysts, according to what is described by Dehmlow and Dehmlow in "Phase Transfer Catalysis" (1983), Verlag Chemie.

The intermediates having general formulae (II), (III), (IV), (V), (VI) and (VII), when not already known in themselves, can be easily prepared according to methods known in organic chemical practice.

As already mentioned, the compounds having general formula (I) have a high herbicidal activity which makes them suitable for use in the agrarian field to protect useful crops from weeds.

In particular, the compounds object of the present invention are effective in both the pre-emergence and post-emergence control of numerous monocotyledon and dicotyledon weeds. At the same time, these compounds show compatibility or the absence of toxic effects with respect to useful crops in pre- and/or post-emergence treatment.

Examples of weeds which can be effectively controlled using the compounds having general formula (I) are: Abutilon theofrasti, Alisma plantago, Amaranthus spp., Amni maius, Capsella bursa pastoris, Chenopodium album, Convolvulus sepium, Galium aparine, Geranium dissectum, Ipomea spp., Matricaria spp., Papaver rhoeas, Phaseolus aureus, Polygonum persicaria, Portulaca oleracea, Sida spinosa, Sinapis arvensis, Solanum nigrum, Stellaria media, Veronica spp., Viola spp., Xanthium spp., Alopecurus myosuroides, Avena fatua, Cyperus spp., Digitaria sanguinalis, Echinocloa spp., Heleocaris avicularis, Heteranthera spp., Panicum spp., Poa spp., Scirpus spp., Sorghum spp., etc.

With the doses used for agrarian applications, the above compounds have not shown any toxic effects towards any of the important agrarian crops such as rice (Oryza sativa), wheat (Triticum sp.), barley (Hordeum vulgare), maize (Zea mays), soybean (Glycine max).

A further object of the present invention relates to a method for controlling weeds in cultivated areas by the application of the compounds having general formula (I).

The quantity of compound to be applied for obtaining the desired effect can vary in relation to various factors such as, for example, the compound used, the crop to be preserved, the weed to be destroyed, the degree of infestation, the climatic conditions, the characteristics of the ground, the application method, etc.

Doses of compound ranging from 1 g to 1000 g per hectare generally provide sufficient control.

For use in agriculture, it is often advantageous to use compositions with a herbicidal activity containing, as active substance, one or more compounds having general formula (I), also possibly as a mixture of isomers.

Compositions can be used, in the form of dry powders, wettable powders, emulsifiable concentrates, microemulsions, pastes, granulates, solutions, suspensions, etc.: the selection of the type of composition depends on the specific use.

The compositions are prepared according to the known methods, for example by diluting or dissolving the active substance with a solvent and/or solid diluent medium, optionally in the presence of surface-active agents.

Kaolin, alumina, silica, talc, bentonite, chalk, quartz, dolomite, attapulgite, montmorillonite, diatomaceous earth, cellulose, starch, etc., can be used as solid inert diluents, or carriers.

Water, or organic solvents such as aromatic hydrocarbons (xylols, mixtures of alkylbenzols, etc.), aliphatic hydrocarbons (hexane, cyclohexane, etc.), halogenated aromatic hydrocarbons (chlorobenzol, etc.), alcohols (methanol, propanol, butanol, octanol, etc.), esters (isobutyl acetate, etc.), ketones (acetone, cyclohexanone, acetophenone, isophorone, ethylamylketone, etc.), or vegetable or mineral oils or their mixtures, etc., can be used as liquid inert diluents.

Wetting and emulsifying agents of the non-ionic type (polyethoxylated alkylphenols, polyethoxylated fatty alcohols, etc.), of the anionic type (alkylbenzenesulfonates, alkylsulfonates, etc.), of the cationic type (quaternary salts of alkylammonium, etc.), can be used as surface-active agents.

Dispersing agents (for example lignin and its salts, derivatives of cellulose, alginates, etc.), stabilizers (for example antioxidants, ultraviolet-ray absorbers, etc.), can also be added.

In order to broaden the action range of the above compositions, it is possible to add other active ingredients such as, for example, other herbicides, fungicides, insecticides, acaricides, fertilizers, etc.

Examples of other herbicides which can be added to the compositions containing one or more compounds having general formula (I) are the following:
acetochlor, acifluorfen, aclonifen, AKH-7088, alachlor, alloxydim, ametryn, amicarbazone, amidosulfuron, amitrole, anilofos, asulam, atrazine, azafenidin, azimsulfuron, aziprotryne, BAY MKH 6561, beflubutamid, benazolin, benfluralin, benfuresate, bensulfuron, bensulide, bentazone, benzfendizone, benzobicyclon, benzofenap, benzthiazuron, bifenox, bilanafos, bispyribac-sodium, bromacil, bromobutide, bromofenoxim, bromoxynil, butachlor, butafenacil, butamifos, butenachlor, butralin, butroxydim, butylate, cafenstrole, carbetamide, carfentrazone-ethyl, chlomethoxyfen, chloramben, chlorbromuron, chlorbufam, chlorflurenol, chloridazon, chlorimuron, chlornitrofen, chlorotoluron, chloroxuron, chlorpropham, chlorsulfuron, chlorthal, chlorthiamid, cinidon ethyl, cinmethylin, cinosulfuron, clethodim, clodinafop, clomazone, clomeprop, clopyralid, cloransulam-methyl, cumyluron (JC-940), cyanazine, cycloate, cyclosulfamuron, cycloxydim, cyhalofop-butyl, 2,4-D, 2,4-DB, daimuron, dalapon, desmedipham, desmetryn, dicamba, dichlobenil, dichlorprop, dichlorprop-P, diclofop, diclosulam, diethatyl, difenoxuron, difenzoquat, diflufenican, diflufenzopyr, dimefuron, dimepiperate, dimethachlor, dimethametryn, dimethenamid, dinitramine, dinoseb, dinoseb acetate, dinoterb, diphenamid, dipropetryn, diquat, dithiopyr, 1-diuron, eglinazine, endothal, EPTC, esprocarb, ethalfluralin, ethametsulfuron-methyl, ethidimuron, ethiozin (SMY 1500), ethofumesate, ethoxyfen-ethyl (HC-252), ethoxysulfuron, etobenzanid (HW 52), fenoxaprop, fenoxaprop-P, fentrazamide, fenuron, flamprop, flamprop-M, flazasulfuron, florasulam, fluazifop, fluazifop-P, fluazolate (JV 485), flucarbazone-sodium, fluchloralin, flufenacet, flumetsulam, flumiclorac-pentyl, flumioxazin, flumipropin, fluometuron, fluoroglycofen, fluoronitrofen, flupoxam, flupropanate, flupyrsulfuron, flurenol, fluridone, flurochloridone, fluroxypyr, flurtamone, fluthiacet-methyl, fomesafen, foramsulfuron, fosamine, furyloxyfen, glufosinate, glyphosate, halosulfuron-methyl, haloxyfop, haloxyfop-P-methyl, hexazinone, imazamethabenz, imazamox, imazapic, imazapyr, imazaquin, imazethapyr, imazosulfuron, indanofan, iodosulfuron, ioxynil, isopropalin, isoproturon, isouron, isoxaben, isoxachlortole, isoxaflutole, isoxapyrifop, KPP-421, lactofen, lenacil, linuron, LS830556, MCPA, MCPA-thioethyl, MCPB, mecoprop, mecoprop-P, mefenacet, mesotrione, metamitron, metazachlor, methabenzthiazuron, methazole, methoprotryne, methyldymron, metobenzuron, metobromuron, metolachlor, S-metolachlor, metosulam, metoxuron, metribuzin, metsulfuron, molinate, monalide, monolinuron, naproanilide, napropamide, naptalam, NC-330, neburon, nicosulfuron, nipyraclofen, norflurazon, orbencarb, oryzalin, oxadiargyl, oxadiazon, oxasulfuron, oxaziclomefone, oxyfluorfen, paraquat, pebulate, pendimethalin, pentanochlor, pentoxazone, pethoxamid, phenmedipham, picloram, picolinafen, piperophos, pretilachlor, primisulfuron, prodiamine, profluazol, proglinazine, prometon, prometryne, propachlor, propanil, propaquizafop, propazine, propham, propisochlor, propyzamide, prosulfocarb, prosulfuron, pyraclonil, pyraflufenethyl, pyrazolynate, pyrazosulfuron, pyrazoxyfen, pyribenzoxim, pyributicarb, pyridafol, pyridate, pyriftalid, pyriminobac-methyl, pyrithiobac-sodium, quinclorac, quinmerac, quizalofop, quizalofop-P, rimsulfuron, sethoxydim, siduron, simazine, simetryn, sulcotrione, sulfentrazone, sulfometuron-methyl, sulfosulfuron, 2,3,6-TBA, TCA-sodium, tebutam, tebuthiuron, tepraloxydim, terbacil, terbumeton, terbuthyl-azine, terbutryn, thenylchlor, thiazafluron, thiazopyr, thidiazimin, thifensulfuron-methyl, thiobencarb, tiocarbazil, tioclorim, tralkoxydim, tri-allate, triasulfuron, triaziflam, tribenuron, triclopyr, trietazine, trifloxysulfuron, trifluralin, triflusulfuron-methyl, tritosulfuron, UBI-C4874, vernolate.

The concentration of active substance in the above compositions can vary within a wide range, depending on the active compound, the applications for which they are destined, the environmental conditions and the type of formulation used.

In general, the concentration of active substance preferably ranges from 1 to 90%.

Some illustrative and non-limiting examples of the present invention are provided hereunder.

### EXAMPLE 1

### Preparation of N-(4-methyl-2-(3-trifluoromethylphenyl)-methoxyphenyl]cyclopropanecarboxamide (C-1).

A solution of N-(2-hydroxy-4-methylphenyl)cyclopropanecarboxamide (1g; 5.2 mmoles) in N,N-dimethylformamide (DMF; 13 ml), is prepared in a 100 ml flask, maintained under an atmosphere of nitrogen. Potassium carbonate is added (1 g; 7.2 mmoles), the mixture is kept under stirring at room temperature and a solution of 3-trifluoromethylbenzyl chloride (1.1 g; 5.6 mmoles) in DMF (2 ml) is added. The mixture is heated to 60°C for 3 hours under stirring. After cooling, it is poured into water (150 ml), extracted with ethyl acetate (3 x 50 ml), the organic phase is washed with water (2 x 50 ml), anhydrified with sodium sulfate, filtered and concentrated. The raw product (1.6 g) is crystallized from a mixture of hexane/ethyl acetate 8:2: 1.3 g of a crystalline solid are obtained, with a melting point at 153-155°C.

### EXAMPLE 2

### Preparation of N-[4-methyl-2-(3-trifluoromethylphenoxy)-methylphenyl]cyclopropanecarboxamide (C-2).

A solution of cyclopropanecarbonyl chloride (0.54 g; 5.16 mmoles) in methylene chloride (5 ml) is added dropwise - at 0°C and under stirring - to a mixture of 4-methyl-2-trifluoromethyl-phenoxymethyl)aniline (1.4 g; 5 mmoles) and triethylamine (0.55 g; 5.44 mmoles) in methylene chloride (15 ml) in a 100 ml flask, maintained under an atmosphere of nitrogen. The mixture is heated to reflux temperature for 3 hours under stirring. After cooling, the mixture is poured into water (150 ml), extracted with methylene chloride (3 x 50 ml), the organic phase is washed with water (2 x 50 ml), anhydrified with sodium sulfate, filtered and concentrated. 1.5 g of the desired product are obtained (¹H-NMR, elemental analysis).

### EXAMPLE 3

Operating analogously to what is described in Examples 1 and 2 above, the compounds (C) having general formula (I) indicated in Table 2 below, were prepared.

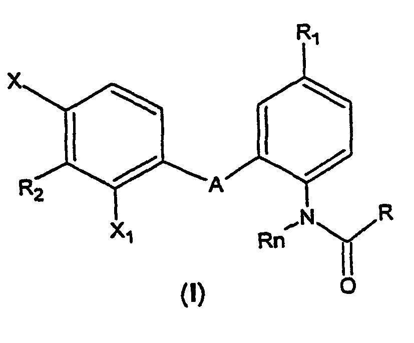

**Table 2**

| C | X | X₁ | R₂ | A | R₁ | Rₙ | R | M.P. (°C) |
|---|---|---|---|---|---|---|---|---|
| 1 | H | H | CF₃ | CH₂O | CH₃ | H | cyclopropyl | 153-155 |
| 2 | H | H | CF₃ | CH₂O | CH₃ | H | ClCH₂ | |
| 3 | H | H | CF₃ | CH₂O | CH₃ | H | H | |
| 4 | H | H | OCF₂H | CH₂O | CH₃ | H | cyclopropyl | |
| 5 | H | H | CF₃ | CH₂O | CH₃ | H | 1-methylcyclopropyl | |
| 6 | H | H | CF₃ | CH₂O | CH₃ | H | cyclobutyl | |
| 7 | H | H | CF₃ | CH₂O | CH₃ | H | cyclopentyl | |
| 8 | H | H | CF₃ | CH₂O | CH₃ | H | CH₃OCH₂ | |
| 9 | H | H | CF₃ | CH₂O | CH₃ | H | (CH₃)₂N | |
| 10 | H | H | CF₃ | CH₂O | CH₃ | H | 1-pyrrolidyl | |
| 11 | H | H | CF₃ | CH₂O | CH₃ | H | 1-morpholyl | |
| 12 | H | H | CF₃ | CH₂O | F | H | cyclopropyl | |
| 13 | H | H | CF₃ | CH₂O | CH₃ | H | CH₃O | |
| 14 | H | H | CF₃ | CH₂O | CH₃ | H | CH₃CH₂O | |
| 15 | H | F | CF₃ | CH₂O | CH₃ | H | cyclopropyl | |

### EXAMPLE 4

### Determination of the herbicidal activity and phytotoxicity in pre-emergence.

The herbicidal activity of the compounds of the invention in pre-emergence was evaluated according to the following operating procedure.

The vegetable species of interest (weeds or crops) were planted in vases having a diameter of over 10 cm, a height of 10 cm and containing sandy soil. 10 vases were used for each vegetable species.

The vases were divided into a further two groups each containing 5 vases for each weed or crop.

24 hours after planting, the vases were dampened with a light shower. An hour after wetting, the first group of vases was treated with a hydro-acetone dispersion containing acetone at 10% v, the product to be evaluated at the desired concentration and Tween 20 at 0.5%.

The second group was only treated with a hydro-acetone solution containing acetone at 10% by volume and Tween 20 at 0.5%, and was used as a comparison (blank).

After the treatment, all the vases were uniformly watered and kept in a conditioned environment under the following environmental conditions:
- temperature: 24°C
- relative humidity: 60%
- photoperiod: 16 hours
- light intensity: 10,000 lux

28 days after treatment, the herbicidal activity was evaluated on the basis of the following scale of values referring to the percentage of damage measured on the treated plants with respect to those not treated (blank):
- 0 = 0-9% damage
- 1 = 10-29% damage
- 2 = 30-49% damage
- 3 = 50-69% damage
- 4 = 70-89% damage
- 5 = 90% damage - death of the plant

Table 3 indicates the results obtained with compound C-1, evaluated at a dose of 500 g/ha on the following vegetable species:
Amarantus retroflexus (AR), Capsella bursa pastoris (CP), Chenopodium album (CA), Galium aparine (GA), Matricaria chamomilla (MC), Papaver rhoeas (PR), Portulaca oleracea (PO), Stellaria media (SM), Alopecurus myosuroides (AM), Panicum dicothomiflorum (PD), maize (M), wheat (F).

**Table 3: Herbicidal activity in pre-emergence at a dose of 500 g/ha**

| Vegetable → | **AR** | **CP** | **CA** | **GA** | **MC** | **PR** | **PO** | **SM** | **AM** | **PD** | **M** | **F** |
|---|---|---|---|---|---|---|---|---|---|---|---|---|
| ↓Compound | | | | | | | | | | | | |
| **C-1** | 5 | 5 | 5 | 5 | 5 | 5 | 5 | 5 | 5 | 5 | 0 | 0 |

## Claims

1. Compounds having general formula (I): 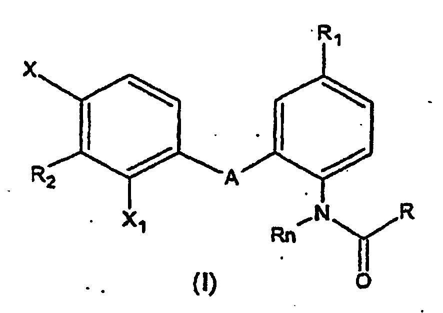 wherein:
- R represents a hydrogen atom, a C₁-C₈ haloalkyl group, a C₂-C₈ alkoxyalkyl or haloalkoxyalkyl group, a C₂-C₈ alkenyl or haloalkenyl group, a C₂-C₈ alkynyl or haloalkynyl group, a C₃-C₈ cycloalkyl or C₄-C₉ cycloalkylalkyl group optionally substituted by halogen atoms and/or C₁-C₄ alkyl or haloalkyl groups, an NRₐR_{b} group;
- Rₐ and R_{b}, the same or different, represent: a hydrogen atom; a C₁-C₈ alkyl group; a phenyl group or a benzyl group optionally substituted by halogen atoms, by CN groups, NO₂ groups, C₁-C₄ alkyl, haloalkyl, alkoxyl, haloalkoxyl groups; or Rₐ and R_{b} jointly represent a C₂-C₈ alkylene chain optionally interrupted by oxygen atoms;
- Rₙ represents a hydrogen atom, or a C₁-C₄ alkyl or haloalkyl group;
- R₁ represents a halogen atom, a C₁-C₄ alkyl or haloalkyl group, a C₁-C₄ alkoxyl or haloalkoxyl group, a C₁-C₄ alkylthio or haloalkylthio group, a cyano group, a nitro group;
- R₂ represents a C₁-C₄ haloalkyl or haloalkoxyl group;
- X and X₁, the same or different, represent a hydrogen atom or a halogen atom;
- A represents a -CR₃R₄O-group, wherein R₃ and R₄, the same or different, represent a hydrogen atom or a C₁-C₄ alkyl, haloalkyl group.

2. A process for the preparation of the compounds having general formula (I) wherein an amine derivative having general formula (II) is reacted with a compound having general formula (III), according to reaction scheme 1: 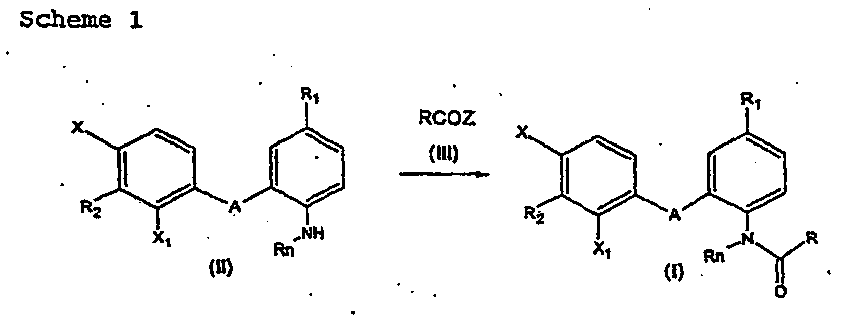 wherein R, Rₙ, R₁, R₂, X, X₁ and A have the meanings described above, Z represents a halogen atom, an alkoxyl groups a hydroxyl group.

3. The process according to claim 2, wherein Z represents a halogen atom and the reaction is carried out in an inert solvent and in the presence of an organic or inorganic base at a temperature ranging from -20°C to the boiling point of the reaction mixture.

4. A process for the preparation of compound having general formula (I) wherein A represents a -CR₃R₄O-group comprising the condensation of a derivative having general formula (IV) with a phenol having general formula (V), to give an ether having general formula (Ia) according to reaction scheme 2: 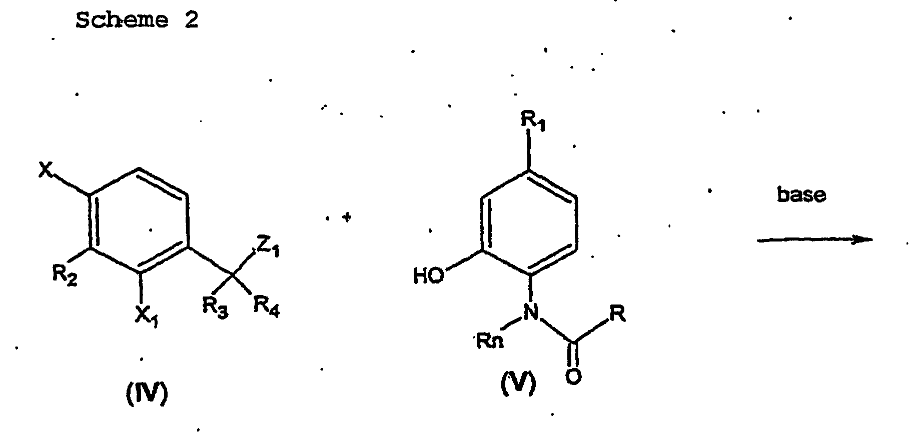 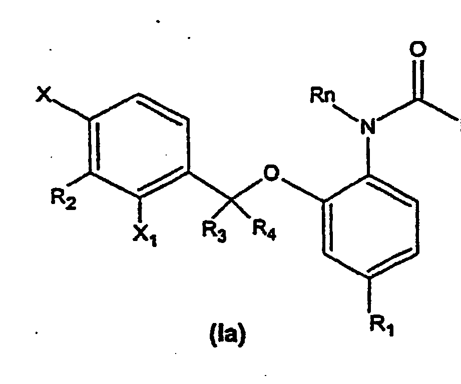 wherein R, Rₙ, R₁, R₂, R₃, R₄, X and X₁ have the meanings previously defined, Z₁ represents a halogen atom, or an R_{z}SO₃ group wherein R_{z} represents a C₁-C₄ alkyl group or a phenyl group optionally substituted by C₁-C₄ alkyl groups, and the reaction is carried out in the presence of one or more inert organic solvents and a base, at a temperature ranging from -10°C to the boiling point of the reaction mixture.

5. Use of the compounds according to claim 1 as herbicides.

6. Herbicidal compositions comprising solid carriers, liquids diluents, surface-active agents or other special additives and at least one of the compounds according to claim 1.

7. The compositions according to the previous claim, wherein the concentration of the compounds ranges from 1 to 90%.

8. The compositions according to claim 7 also comprising other herbicides.

9. A method for controlling weeds in cultivated areas which consists in applying a compound according to claim 1 or a composition according to claim 6, to said areas.

10. The method according to claim 9, wherein the active compounds are applied at doses ranging from 1 g to 1000 g per hectare.

## Patentansprüche

1. Verbindungen der allgemeinen Formel (I): 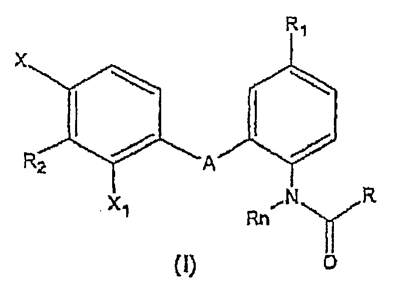 worin:
- R ein Wasserstoffatom, eine C₁-C₈-Halogenalkylgruppe, eine C₂-C₈-Alkoxyalkyl- oder -Halogenalkoxyalkylgruppe, eine C₂-C₈-Alkenyl- oder -Halogenalkenylgruppe, eine C₂-C₈-Alkinyl- oder -Halogenalkinylgruppe, eine C₃-C₈-Cycloalkyl- oder C₄-C₉-Cycloalkylalkylgruppe, gegebenenfalls substituiert mit Halogenatomen und/oder C₁-C₄-Alkyl- oder -Halogenalkylgruppen; oder eine NRₐR_{b}-Gruppe ist;
- Rₐ und R_{b}, die gleich oder verschieden sind, ein Wasserstoffatom, eine C₁-C₈-Alkylgruppe; eine Phenylgruppe oder eine Benzylgruppe, gegebenenfalls substituiert mit Halogenatomen, mit CN-Gruppen, NO₂-Gruppen, C₁-C₄-Alkyl-,
- Halogenalkyl-, -Alkoxyl-, -Halogenalkoxylgruppen, sind; oder Rₐ und R_{b} gemeinsam eine C₂-C₈-Alkylenkette sind, gegebenenfalls unterbrochen durch Sauerstoffatome;
- Rₙ ein Wasserstoffatom oder eine C₁-C₄-Alkyl- oder -Halogenalkylgruppe ist;
- R₁ ein Halogenatom, eine C₁-C₄-Alkyl- oder -Halogenalkylgruppe, eine C₁-C₄-Alkoxyl- oder -Halogenalkoxylgruppe, eine C₁-C₄-Alkylthio- oder -Halogenalkylthiogruppe, eine Cyanogruppe, eine Nitrogruppe ist;
- R₂ eine C₁-C₄-Halogenalkyl- oder -Halogenalkoxylgruppe ist;
- X und X₁, die gleich oder verschieden sind, ein Wasserstoffatom oder ein Halogenatom sind;
- A eine Gruppe -CR₃R₄O- ist, worin R₃ und R₄, die gleich oder verschieden sind, ein Wasserstoffatom oder eine C₁-C₄-Alkyl-, -Halogenalkylgruppe sind.

2. Verfahren zur Herstellung der Verbindungen der allgemeinen Formel (I), wobei ein Aminderivat der allgemeinen Formel (II) umgesetzt wird mit einer Verbindung der allgemeinen Formel (III), gemäß dem Reaktionsschema 1: 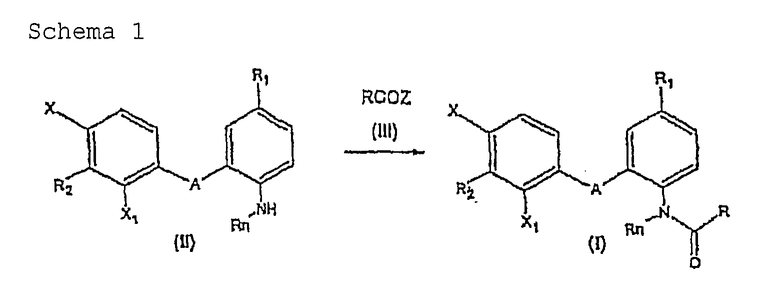 worin R, Rₙ, R₁, R₂, X, X₁ und A die vorstehend angegebenen Bedeutungen haben, Z ein Halogenatom, eine Alkoxylgruppe, eine Hydroxylgruppe ist.

3. Verfahren nach Anspruch 2, worin Z ein Halogenatom ist und die Umsetzung in einem inerten Lösungsmittel und in Gegenwart einer organischen oder anorganischem Base bei einer Temperatur im Bereich von -20°C bis zum Siedepunkt des Reaktionsgemisches durchgeführt wird.

4. Verfahren zur Herstellung von Verbindungen der allgemeinen Formel (I), worin A eine Gruppe -CR₃R₄O- ist, umfassend die Kondensation eines Derivats der allgemeinen Formel (IV) mit einem Phenol der allgemeinen Formel (V), um einen Ether der allgemeinen Formel (Ia) zu erhalten, gemäß dem Reaktionsschema 2: 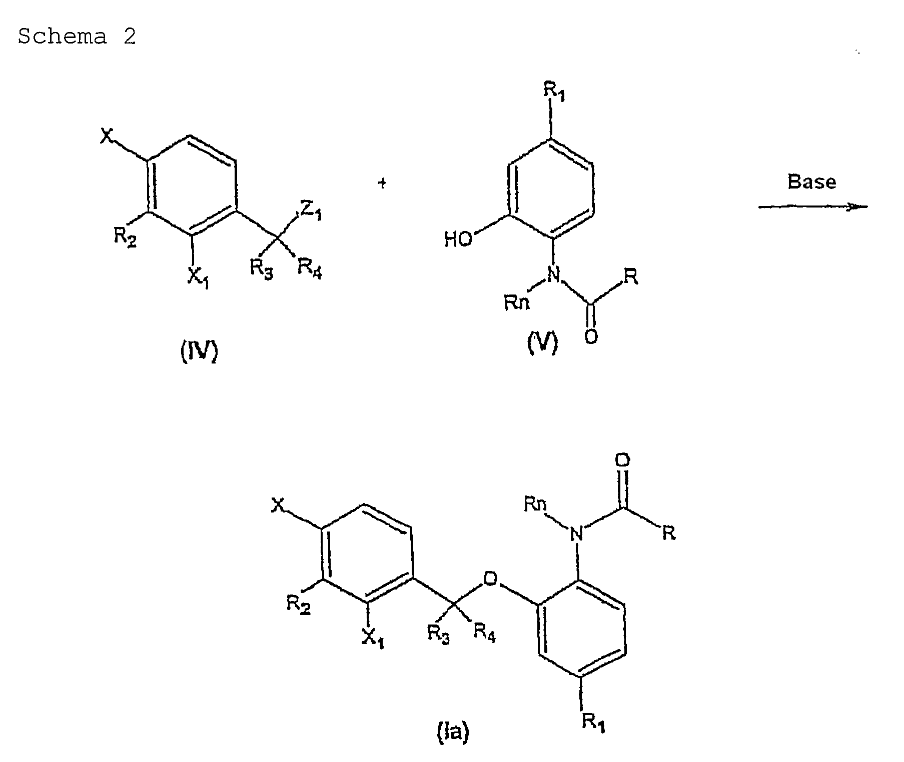 worin R, Rₙ, R₁, R₂, R₃, R₄, X und X₁ die vorstehend angegebenen Bedeutungen haben, Z₁ ein Halogenatom oder eine Gruppe R_{z}SO₃- ist, worin R_{z} eine C₁-C₄-Alkylgruppe oder eine Phenylgruppe ist, gegebenenfalls substituiert mit C₁-C₄-Alkylgruppen, und die Umsetzung ausgeführt wird in Gegenwart von einem oder mehreren inerten organischen Lösungsmitteln und einer Base, bei einer Temperatur im Bereich von -10°C bis zum Siedepunkt des Reaktionsgemisches.

5. Verwendung der Verbindungen gemäß Anspruch 1 als Herbizide.

6. Herbizidzusammensetzungen, umfassend feste Trägerstoffe, flüssige Verdünnungsmittel, oberflächenaktive Mittel oder andere Spezialadditive und mindestens eine der Verbindungen gemäß Anspruch 1.

7. Zusammensetzungen gemäß dem vorstehenden Anspruch, worin die Konzentration der Verbindungen im Bereich von 1 bis 90% liegt.

8. Zusammensetzungen nach Anspruch 7, die außerdem weitere Herbizide umfassen. ,

9. Verfahren zur Kontrolle bzw. Bekämpfung von Unkraut in kultivierten Bereichen, bestehend aus der Anwendung einer Verbindung gemäß Anspruch 6, oder einer Zusammensetzung gemäß Anspruch 6 auf diese Bereiche.

10. Verfahren nach Anspruch 9, wobei die wirksamen Verbindungen angewendet werden in Dosen im Bereich von 1 g bis 1000 g pro Hektar.

## Revendications

1. Composés répondant à la formule générale (I) : 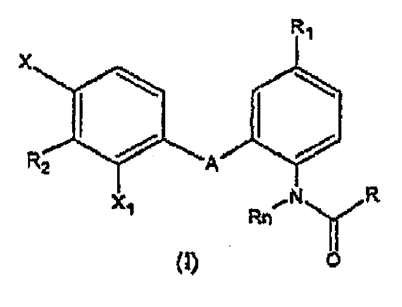 dans laquelle :
- R représente un atome d'hydrogène, un groupe halogénoalkyle en C₁ à C₈, un groupe alcoxyalkyle ou halogénoalcoxyalkyle en C₂ à C₈, un groupe alcényle ou halogénoalcényle en C₂ à C₈, un groupe alcynyle ou halogénoalcynyle en C₂ à C₈, un groupe cycloalkyle en C₃ à C₈ ou cycloalkylalkyle en C₄ à C₉ facultativement substitué par des atomes d'halogène et/ou des groupes alkyle ou halogénoalkyle en C₁ à C₄, un groupe NRₐR_{b},
- Rₐ et R_{b}, identiques ou différents, représentent : un atome d'hydrogène ; un groupe alkyle en C₁ à C₈ un groupe phényle ou un groupe benzyle facultativement substitué par des atomes d'halogène, par des groupes CN, des groupes NO₂, des groupes alkyle en C₁ à C₄, halogénoalkyle, alcoxyle, halogénoalcoxyle; ou bien Rₐ et R_{b} représentent conjointement une chaîne alkylène en C₂ à C₈ facultativement interrompue par des atomes d'oxygène ;
- Rₙ représente un atome d'hydrogène, ou un groupe alkyle ou halogénoalkyle en C₁ à C₄ ;
- R₁ représente un atome d'halogène, un groupe alkyle ou halogénéoalkyle en C₁ à C₄, un groupe alcoxyle ou halogénoalcoxyle en C₁ à C₄, un groupe alkylthio ou halogénoalkylthio en C₁ à C₄, un groupe cyano, un groupe nitro ;
- R₂ représente un groupe halogénoalkyle ou halogénoalcoxyle en C₁ à C₄;
- X et X₁, identiques ou différents, représentent un atome d'hydrogène ou un atome d'halogène ;
- A représente un groupe -CR₃R₄O-, dans lequel R₃ et R₄, identiques ou différents, représentent un atome d'hydrogène ou un groupe alkyle ou halogénoalkyle en C₁ à C₄.

2. Procédé de préparation des composés répondant à la formule générale (I), dans lequel un dérivé amine répondant à la formule générale (II) réagit avec un composé répondant à la formule générale (III), selon le schéma de réaction 1 : 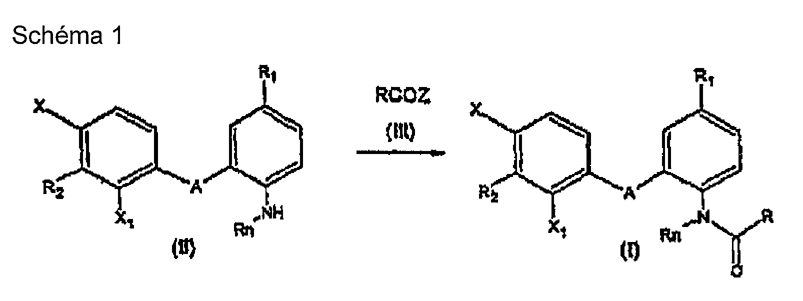 dans lequel R, Rₙ, R₁, R₂, X, X₁ et A ont les significations décrites ci-dessus, Z représente un atome d'halogène, un groupe alcoxyle ou un groupe hydroxyle.

3. Procédé selon la revendication 2, dans lequel Z représente un atome d'halogène et la réaction est réalisée dans Un solvant inerte et en présence d'une base organique ou inorganique à une température variant de -20 °C au point d'ébullition du mélange de réaction.

4. Procédé de préparation de composés répondant à la formule générale (1) dans laquelle A représente un groupe -CR₃R₄O- comprenant la condensation d'un dérivé répondant à la formule générale (IV) avec un phénol répondant à la formule générale (V), pour donner un éther répondant à la formule générale (la) selon le schéma de réaction 2 : 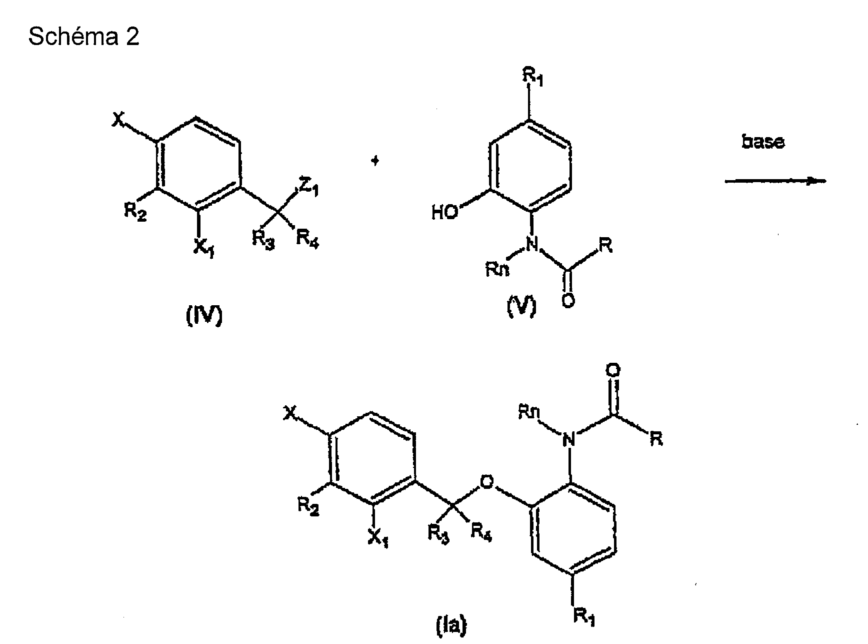 dans lequel R, Rₙ, R₁, R₂, R₃, R₄, X et X₁ ont les significations définies précédemment, Z₁ représente un atome d'halogène, ou un groupe R_{z}SO₃ dans lequel R_{z} représente un groupe alkyle en C₁ à C₄ ou un groupe phényle facultativement substitué par des groupes alkyle en C₁ à C₄, et la réaction est réalisée en présence d'un ou plusieurs solvants organiques inertes et d'une base, à une température variant de -10 °C au point d'ébullition du mélange de réaction.

5. Utilisation des composés selon la revendication 1, comme herbicides.

6. Compositions herbicides comprenant des supports solides, des diluants liquides, des agents tensioactifs ou d'autres additifs spéciaux et au moins l'un des composés selon la revendication 1.

7. Compositions selon la revendication précédente, dans lesquelles la concentration des composés varie de 1 à 90 %.

8. Compositions selon la revendication 7, comprenant également d'autres herbicides.

9. Procédé pour lutter contre les mauvaises herbes dans des zones cultivées qui consiste à appliquer un composé selon la revendication 1 ou une composition selon la revendication 6, auxdites zones.

10. Procédé selon la revendication 9, dans lequel les composés actifs sont appliqués à des doses variant de 1 g à 1 000 g par hectare.
